# EUROPEAN PATENT SPECIFICATION

(11) **EP 2 866 024 B1**
(45) Date of publication and mention of the grant of the patent: **18.03.2020**
(21) Application number: 13808596.4
(22) Date of filing: 25.06.2013
(51) Int. Cl.: G01N 27/327, C12Q 1/00

(54) **ENZYME ELECTRODE**
ENZYMELEKTRODE
ÉLECTRODE ENZYMATIQUE

(30) Priority: 25.06.2012 JP 2012142376
(43) Date of publication of application: 29.04.2015
(73) Proprietor: BioEngineering Laboratories, LLC, Tokyo 160-0004 (JP)
(72) Inventor: TSUKADA, Masashi, Kyoto-shi, Kyoto 602-0008 (JP); TAJIMA, Mayumi, Kyoto-shi, Kyoto 602-0008 (JP)
(74) Representative: Dehns
(86) International application number: PCT/JP2013/067382
(87) International publication number: WO 2014/002999

(56) References cited:
- WO-A1-2009/133679
- JP-A- H0 299 849
- JP-A- H04 122 849
- JP-A- H09 127 041
- JP-A- 2004 163 385
- JP-A- 2014 006 155
- US-A1- 2007 267 301
- US-A1- 2009 321 277
- GÃBEL G ET AL: "Direct electron transfer of PQQ-glucose dehydrogenase at modified carbon nanotubes electrodes", ELECTROCHEMISTRY COMMUNICATIONS, vol. 13, no. 11, 22 August 2011 (2011-08-22), pages 1240-1243, XP028320515, ISSN: 1388-2481, DOI: 10.1016/J.ELECOM.2011.08.034 [retrieved on 2011-08-28]
- OKUDA J ET AL: "PQQ glucose dehydrogenase with novel electron transfer ability", BIOCHEMICAL AND BIOPHYSICAL RESEARCH COMMUNICATIONS, vol. 314, no. 3, 13 February 2004 (2004-02-13), pages 793-797, XP004485036, ISSN: 0006-291X, DOI: 10.1016/J.BBRC.2003.12.167
- YAMAZAKI T ET AL: "Construction and characterization of direct electron transfer-type continuous glucose monitoring system employing thermostable glucose dehydrogenase complex", ANALYTICAL LET, TAYLOR & FRANCIS INC, US, vol. 41, no. 13, 1 January 2008 (2008-01-01), pages 2363-2373, XP008124724, ISSN: 0003-2719, DOI: 10.1080/00032710802350567
- Hitomi Shimizu ET AL: "Glucose Monitoring by Direct Electron Transfer Needle-Type Miniaturized Electrode", Electrochemistry, vol. 80, no. 5, 1 January 2012 (2012-01-01), pages 375-378, XP055378548, JP ISSN: 1344-3542, DOI: 10.5796/electrochemistry.80.375

## Description

### Technical Field

The present invention relates to an enzyme electrode.

### Background Art

There is known an enzyme electrode including an electrode and a detection layer containing an enzyme that is formed on the electrode. The enzyme electrode has a structure that takes electrons generated by an enzyme reaction out of the electrode.

### Prior Art Documents

### Patent Document

Patent Document 1: Japanese Laid-Open Patent Publication No. 01-075956
Patent Document 2: Japanese Laid-Open Patent Publication No. 63-050748
Patent Document 3: Japanese Laid-Open Patent Publication No. 10-100306
Patent Document 4: Japanese Laid-Open Patent Publication No. 2006-234788

### Non Patent Document

Non Patent Document 1: Brianna C. Thompson et al., Macromol. Rapid Commun. 2010, 31, 1293-1297
Non Patent Document 2: A. Kros et al., Third generation Polyethylenedioxythiophene (PEDOT) based glucose sensor, Symposia Papers Presented Before the Division of Environmental Chemistry, American Chemical Society Anaheim, CA March 21-25, 1999
Non Patent Document 3: A. Kros et al., Poly(pyrrole) versus poly(3,4-ethylenedioxythiophene): implications for biosensor applications, Sensors and Actuators B 106 (2005) 289-295
Non Patent Document 4: C. G. J. Koopal et al., Glucose sensor utilizing polypyrrole incorporated in track-etch membranes as the mediator, Biosensor and Bioelectronics 7 (1992) 461-471
Non Patent Document 5: G. Göbel et al., Direct electron transfer of PQQ-glucose dehydrogenase at modified carbon nanotubes electrodes, Electrochemistry Communications 13 (2011) 1240-1243
Non Patent Document 6: H. Shimizu et al., Glucose Monitoring by Direct Electron Transfer Needle-Type Miniaturized Electrode, Electrochemistry 80 (2012) 375-378

### Summary of the Invention

### Problems to be solved by the Invention

Generally, when an enzyme contained in a detection layer of an enzyme electrode is in an inactive state, the function as an enzyme electrode will be impaired. One of the factors of the inactivation of an enzyme is the inactivation by heat. For example, heat leading to the inactivation may be applied to an enzyme electrode in an environment during storage or transportation. Further, heat may be applied while using an enzyme electrode. It is preferred that an enzyme electrode have good heat resistance in a sense of increasing the life of an enzyme electrode.

One aspect of the present invention is directed to provide an enzyme electrode having improved heat resistance.

### Means for solving the Problems

In order to achieve an object as mentioned above, one aspect of the present invention applies configurations below. Namely, the one aspect of the present invention is an enzyme electrode. The enzyme electrode includes an electrode, and a detection layer that is in contact with the electrode and includes an oxidoreductase, a water-soluble conductive polymer, and conductive particles, wherein electrons are transferred between the enzyme and the electrode by direct electron transfer in the detection layer. According to the invention, the water-soluble conductive polymer is polyaniline sulfonic acid.

In the one aspect of the present invention, the oxidoreductase is a glucose dehydrogenase containing a cytochrome subunit and is surrounded by the polymer. Further, the oxidoreductase also includes a catalytic subunit.

The catalytic subunit may include at least one selected from pyrroloquinoline quinone and flavin adenine dinucleotide.

In the one aspect of the present invention, the conductive particles may include carbon. For example, the concentration of the above-described polyaniline sulfonic acid in a solution to be applied to the electrode is 0.01 to 2%. A functional group of the above-mentioned polyaniline sulfonic acid may be a hydroxy group or a sulfo group.

Also the electrode may include conductive particles.

The enzyme electrode in the one aspect of the present invention is characterized in that it has an improved heat resistance. One of other aspects of the present invention is a biosensor including the enzyme electrode described above.

One of other aspects of the present invention is an electronic apparatus comprising this biosensor.

One of other aspects of the present invention is an apparatus including the enzyme electrode described above, and a feed section that supplies, to a load, a current generated by an enzyme reaction at the enzyme electrode. Further, the other aspects of the present invention may include an electronic apparatus including the above-described apparatus.

One of the other aspects of the present invention is a method of producing an enzyme electrode having improved heat resistance, including forming a detection layer on an electrode, the detection layer including an oxidoreductase, a water-soluble conductive polymer, and conductive particles, wherein the oxidoreductase is surrounded by the polymer, and wherein electrons are transferred between the enzyme and the electrode by direct electron transfer, wherein the oxidoreductase is a glucose dehydrogenase containing a cytochrome subunit; and wherein the water-soluble conductive polymer is polyaniline sulfonic acid.

### Effects of the invention

According to one aspect of the present invention, an enzyme electrode having improved heat resistance may be provided.

### Brief Description of Drawings

[Fig. 1] Fig. 1 is a schematic view illustrating the structure of an enzyme electrode according to the embodiment.
[Fig. 2] Fig. 2 is a schematic view illustrating the inside of a detection layer.
[Fig. 3] Fig. 3 is a graph illustrating the relationship between the temperature and the response current during heat treatment in the case where the time of heat treatment is fixed.
[Fig. 4] Fig. 4 is a graph illustrating the relationship between the time and the response current in the case where the temperature of heat treatment is fixed, and the time of heat treatment is changed.
[Fig. 5] Fig. 5 is a graph illustrating the relationship between the concentration of polyaniline sulfonic acid and the heat resistance.
[Fig. 6] Fig. 6 is a graph illustrating the relationship between the concentration of polyaniline sulfonic acid and the heat resistance.
[Fig. 7] Fig. 7 is a graph illustrating the results of Test 4 (influence of the concentration of a polymer added to a reagent).
[Fig. 8] Fig. 8 is a graph illustrating the results of Test 5 (influence of heat treatment time).

### Description of Embodiments

Hereinafter, an enzyme electrode as an embodiment of the present invention will be described with reference to drawings. The embodiment to be described below is for illustration purposes, and the present invention is not limited to the structure of the following embodiment.

### [Structure of enzyme electrode]

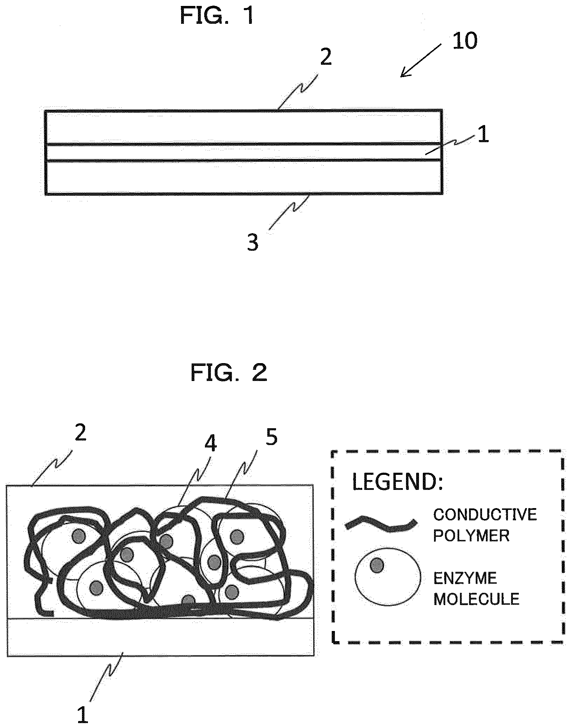

Fig. 1 is a schematic view illustrating the side surface of an enzyme electrode according to the embodiment. In Fig. 1, an enzyme electrode 10 includes an electrode 1 and a detection layer 2 formed on the surface (upper surface in Fig. 1) of the electrode 1.

### <Electrode>

The electrode 1 is formed of a metallic material such as gold (Au), platinum (Pt), silver (Ag), and palladium, or a carbon material such as carbon. The electrode 1 is formed, for example, on an insulating substrate 3 as illustrated in Fig. 1. The insulating substrate 3 is formed of an insulating material such as various resins (plastics) such as a thermoplastic resin, such as polyether imide (PEI), polyethylene terephthalate (PET), and polyethylene (PE), a polyimide resin, and an epoxy resin; glass; ceramics; and paper. Any known material can be applied as an electrode material forming the electrode 1 and a material for the insulating substrate 3. The size and the thickness of the electrode 1 and the insulating substrate 3 can be suitably set. Hereinafter, the combination of the insulating substrate 3 and the electrode 1 may be called a "base material."

### <Detection layer>

Fig. 2 schematically illustrates the state in the detection layer 2 illustrated in Fig. 1. As illustrated in Fig. 2, the detection layer 2 is in contact with the electrode 1, contains the oxidoreductase 4 (hereinafter may be simply represented by an "enzyme 4"), and the conductive polymer (polyaniline sulfonic acid) 5, and does not contain an electron-transfer mediator.

As illustrated in Fig. 2, in the detection layer 2, the molecule of the enzyme 4 has a structure in which it is intricately entangled with the conductive polymer 5. Electrons generated by an enzyme reaction can be transferred to the electrode 1 directly or through the conductive polymer 5. That is, in the enzyme electrode 10 according to the embodiment, electrons are transferred between the enzyme 4 and the electrode 1 by direct electron transfer in the detection layer 2.

Thus, the enzyme electrode 10 is a direct electron transfer-type enzyme electrode. The "direct electron transfer-type enzyme electrode" is a type of enzyme electrode in which electrons are transferred between an enzyme and an electrode in a way in which electrons generated by an enzyme reaction in a detection layer are directly transferred to the electrode without the involvement of an oxidation-reduction material such as an electron-transfer mediator.

Further, the "direct electron transfer-type enzyme electrode" is distinguished from a type of enzyme electrode in which electrons are transferred to an electrode through a material generated by an enzyme reaction, such as the oxidation of hydrogen peroxide (H₂O₂) generated by an enzyme reaction on an electrode when glucose oxidase (GOD) is applied as an enzyme.

Note that, it is said that the maximum limit of distance in which direct electron transfer occurs in a physiological reaction system is 10 to 20 Å. In a longer distance than this maximum limit, in the electron exchange in an electrochemical reaction system including an electrode and a biomolecule (enzyme), as well, it will be difficult to detect the electron exchange on the electrode without the involvement of mass transfer (for example, transfer by diffusion) of the enzyme or a mediator.

In the enzyme electrode 10, entanglement of the molecules of the enzyme 4 and the conductive polymer 5 in the detection layer 2 is considered to act so as to mitigate the action exerted on the enzyme 4 (structural change of the enzyme molecules) by the thermal energy from the outside.

Further, in the detection layer 2, the conductive sites of the conductive polymer 5 are located in the vicinity of the electron transfer sites of the enzyme 4 (sites where electrons are transferred in an enzyme reaction), thereby leading to a state where electron transfer between the enzyme 4 and the electrode 1 easily occurs. As an effect of one aspect of the present invention, the enzyme 4 is probably in an environment where it can easily maintain a higher-order structure of the molecules because the enzyme is surrounded by the conductive polymer 5. Further, electrons generated from the enzyme 4 are probably in a state where a plurality of suitable electron transport pathways from the enzyme 4 to the electrode 1 are formed through the conductive sites of the conductive polymer 5. Therefore, as an effect of one aspect of the present invention, even when the action (physical change or change of arrangement) to the enzyme 4 occurs by thermal energy from the outside, the electron transfer from the enzyme 4 to the electrode 1 is probably large or relieved from a sudden disturbance by having secured the plurality of suitable electron transport pathways. Consequently, the enzyme electrode 10 according to the embodiment can have suitable heat resistance.

Further, in the detection layer 2, the influence of heat stress to the enzyme 4 is probably relieved by thermoelectric conversion characteristics by the Seebeck effect of the conductive polymer (conductive polymer 5). Particularly, as polyaniline sulfonic acid is applied as the conductive polymer 5, the polyaniline sulfonic acid provides a high Seebeck effect because it has a long molecular chain that represents a π-electron conjugated system, has good compatibility (mixing) with the enzyme 4 because it is not water-dispersible but water-soluble, and probably easily structurally entraps the enzyme 4 in the detection layer 2. Therefore, the suppression effect of the influence of heat on the enzyme 4 at least during the preparation of the enzyme electrode 10 is probably improved.

### <<Oxidoreductase>>

The oxidoreductase which is applied as the enzyme 4 is a glucose dehydrogenase containing a cytochrome subunit (CyGDH).

Further, the oxidoreductase contains a catalytic subunit, and the catalytic subunit can have an electron transfer subunit. Examples of the catalytic subunit include an α-subunit of the glucose dehydrogenase containing cytochrome.

The catalytic subunit can contain at least one of pyrroloquinoline quinone (PQQ) and flavin adenine dinucleotide (FAD).

### <<Polyaniline sulfonic acid>>

As the polyaniline sulfonic acid which is applied as the conductive polymer 5, a polyaniline sulfonic acid having various attributes (for example, concerning water solubility) can be applied. The concentration of polyaniline sulfonic acid 6 is, for example, 0.01 to 5%, preferably 0.01 to 2%, by weight in the reagent solution used. Further, a functional group of polyaniline sulfonic acid is a hydroxy group or a sulfo group.

### <<Conductive particles>>

Although not illustrated, the detection layer 2 of the enzyme electrode 10 further contains conductive particles. Since the detection layer 2 contains conductive particles, a more suitable electron transfer to an electrode can be expected. Examples of the conductive particles include carbon. Specifically, metal particles such as gold, platinum, silver, and palladium or a higher-order structure formed from carbon can be applied as the conductive particles. The higher-order structure can include particle or fine particle types of carbon such as conductive carbon black, Ketjen Black, carbon nanotube (CNT), and fullerene. At least one of the metals and carbons as described above can be selected as the conductive particles.

Note that the surface of the detection layer 2 may be covered with an outer-layer film made of cellulose acetate (CA) and the like.

### [Method for preparing enzyme electrode]

The enzyme electrode 10 as described above is prepared, for example, as follows. Specifically, a metal layer which functions as an electrode 1 is formed on one side of an insulating substrate 3. For example, a metal layer having a desired thickness (for example, about 30 nm) is formed by depositing a metallic material by physical vapor deposition (PVD, for example, sputtering) or chemical vapor deposition (CVD) on one side of an insulating substrate 3 in a film form having a predetermined thickness (for example, about 100 µm). An electrode layer formed of a carbon material can also be formed instead of the metal layer.

Next, a detection layer 2 is formed on an electrode 1. That is, a solution (reagent) containing the oxidoreductase 4 containing cytochrome and polyaniline sulfonic acid as the conductive polymer 5 is prepared. The solution (reagent) is dropped on the surface of the electrode 1. The solution (reagent) is dried and solidified on the electrode 1, thereby providing the enzyme electrode 10 in which the detection layer 2 is formed on the electrode 1.

### [Examples]

Hereinafter, Examples of the enzyme electrode will be described.

### <Test 1>

### <<Preparation of reagent solution>>

First, two types of reagent solutions according to Example 1 and Comparative Example as described below were prepared.

### [Example 1]

- Ketjen Black: 0.8%
- Water-soluble polyaniline (trade name: Aqua-PASS (hereinafter represented by "AQP")): 0.45%
- Glucose dehydrogenase containing cytochrome (Cy-GDH): 0.56%
- Stabilizing agent (sucrose): 0.5%
- Phosphate buffer (pH 5.8): 16 mM

Note that "%" represents the percent by weight concentration of a reagent contained in the reagent solution.

### [Comparative Example]

- Ketjen Black: 0.8%
- Glucose dehydrogenase containing cytochrome (Cy-GDH): 0.56%
- Stabilizing agent (sucrose): 0.5%
- Phosphate buffer (pH 5.8): 16 mM

Note that "%" represents the percent by weight concentration of a reagent contained in the reagent solution. Thus, the reagent solution in Comparative Example is a reagent solution in which AQP is removed from the reagent solution in Example 1.

### <<Preparation of enzyme electrode (sample)>>

Next, a plurality of insulating substrates in which an electrode (electrode layer) is formed on one side of the substrate by gold vapor deposition (base materials) were prepared. The reagent solutions according to Example 1 and Comparative Example were each dispensed on each of the insulating substrates, and the resulting base materials were dried by allowing them to stand for 30 minutes in a low humidity drying furnace. In this way, enzyme electrodes (samples) according to Example 1 and enzyme electrodes (samples) according to Comparative Example, in which a detection layer was formed by the solidification of a reagent on each of the electrodes, were obtained.

At this time, four types (a total of eight types) of samples were obtained by drying each of the samples of Example 1 and Comparative Example at a furnace temperature of 30°C, 80°C, 100°C, or 120°C (drying temperature).

### <<Measurement of glucose concentration>>

Next, a response current value was measured for 100 mg/dl of glucose in the above samples after the heat treatment. In the glucose measurement, the above samples were immersed in a phosphate buffer (pH 7.4) heated to 37°C; a platinum wire was used for the counter electrode, and a silver/silver chloride electrode was used for the reference electrode; and the applied voltage to the working electrode was set to +0.4 V (vs. Ag/AgCl).

### <<Evaluation of measurement results>>

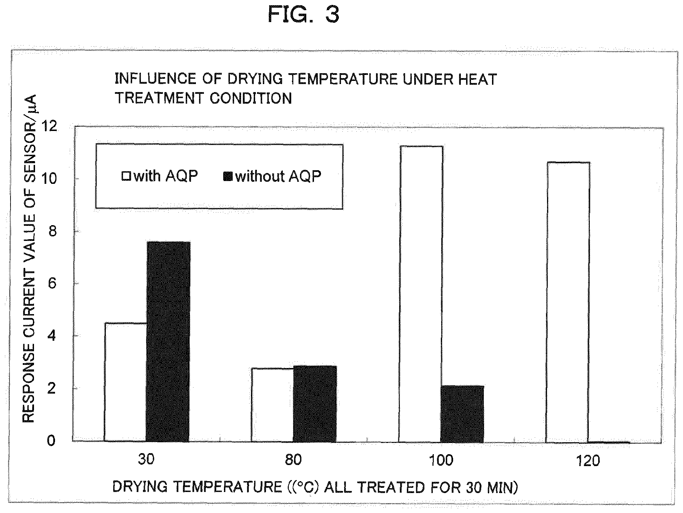

Fig. 3 is a graph illustrating the results of Test 1, that is, the relationship between the response current value (µA) and the drying temperature (°C) for the eight types of samples as described above. In Fig. 3, white bar graphs illustrate the results of the samples according to Example 1, and black bar graphs illustrate the results of the samples according to Comparative Example. Note that the measurement was performed twice using two samples for each type, and the results illustrated in Fig. 3 indicate the average values of the results at the two times of measurement.

According to the test results illustrated in Fig. 3, apparent reduction in response sensitivity was observed with the increase in drying temperature as for the samples to which no AQP was added (Comparative Example), while high response sensitivity was maintained with the increase in drying temperature as for the samples to which AQP was added (Example 1). Thus, it is found that in the samples to which AQP is added, heat resistance of electrode response is largely improved, and improvement in response sensitivity is also achieved.

### <Test 2>

Eight types of reagent solutions according to Example 1 and Comparative Example were prepared in the same manner as in Test 1 as described above, and each reagent solution was dispensed on an electrode in the same base material as the base material used in Test 1. Then, the reagent solution was dried in a low humidity drying furnace (at a drying temperature of 100°C) to obtain an enzyme electrode (sample). However, in Test 2, the drying time (heat treatment time) in the furnace was changed to 10 minutes, 30 minutes, 60 minutes, and 120 minutes to obtain four types (a total of eight types) of enzyme electrodes (samples) for Example 1 and Comparative Example, respectively. Then, the response current values for glucose were measured in the same manner (the measurement techniques and measurement conditions) as in Test 1.

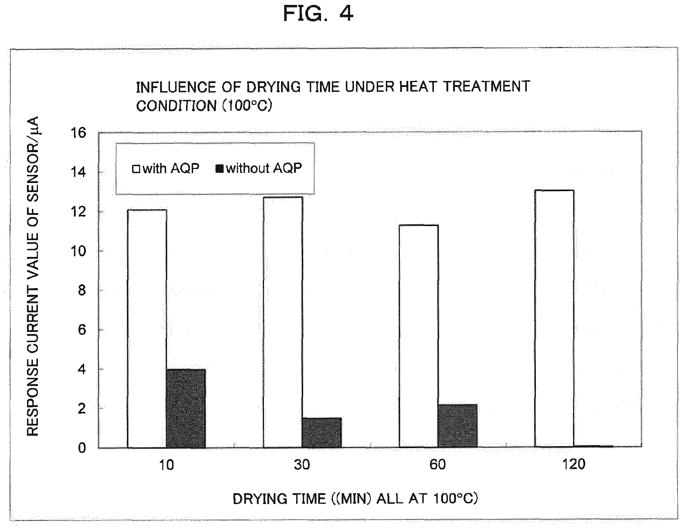

Fig. 4 is a graph illustrating the results of Test 2, that is, the relationship between the response current value (µA) and the drying time (min) for the eight types of samples as described above. In Fig. 4, white bar graphs illustrate the results of the samples according to Example 1, and black bar graphs illustrate the results of the samples according to Comparative Example. Note that the measurement was performed using two samples for each type, and the results illustrated in Fig. 4 indicate the average of the two measurement results.

According to the test results illustrated in Fig. 4, a tendency of significant reduction in response sensitivity was observed with the increase in drying time as for the samples to which no AQP was added (Comparative Example), while a tendency of maintaining suitable response sensitivity was observed irrespective of the change (increase) of drying time as for the samples to which AQP was added (Example 1). Thus, it is found that the samples to which AQP is added can have suitable heat resistance for a long time.

### <Test 3>

Next, four types of reagent solutions in which the concentration of AQP was changed were prepared. The concentration (% by weight) of AQP was set to 0 (Comparative Example), 0.20%, 0.45% (Example 1), and 1%. Note that the components in the reagent solutions excluding AQP were prepared with the same contents as in Example 1.

Four types of reagent solutions obtained in this way were each dispensed on an electrode in the same manner as in Test 1 and dried at each drying time of 10 minutes, 30 minutes, 60 minutes, or 120 minutes (drying temperature was set at 100°C in all cases) to obtain 16 types of enzyme electrodes (samples). Then, the response current value for glucose was measured in the same manner as in Test 1.

### <Results of Test 3>

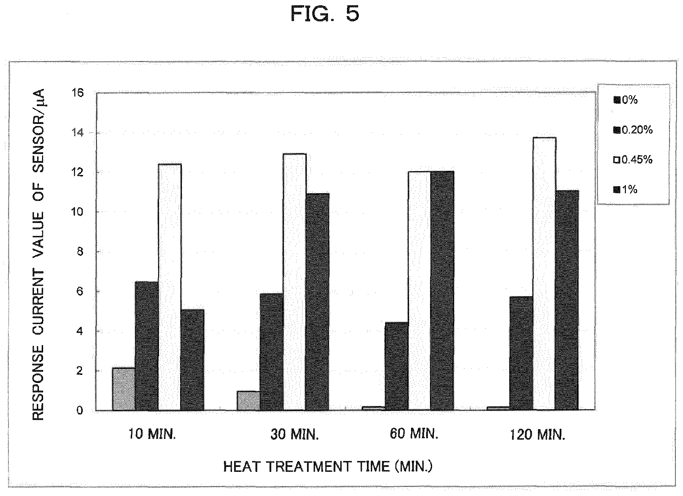

Fig. 5 is a graph illustrating the results of Test 3, that is, the relationship between the drying time (heat treatment time) (minutes) and the response current value (µA) for each sample. In Fig. 5, four bar graphs are illustrated for each heat treatment time. The four bar graphs each represent the response current value (response sensitivity) when the concentration of AQP is 0, 0.20%, 0.45%, or 1%, sequentially from the left.

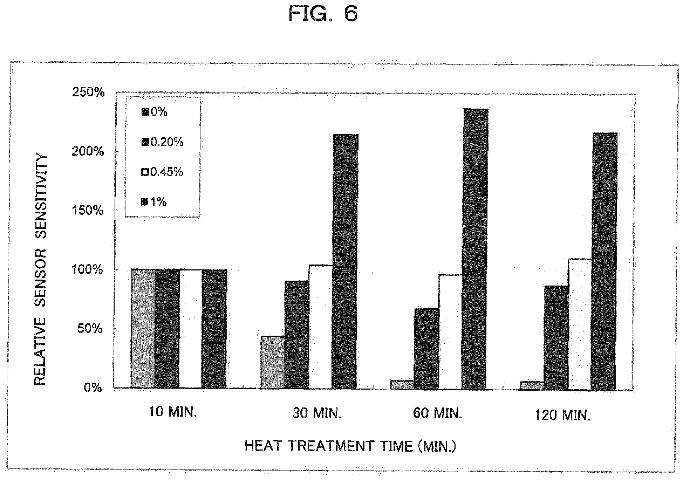

Fig. 6 is a graph illustrating relative response sensitivity (%) at a heat treatment time of 30 minutes, 60 minutes, or 120 minutes when response sensitivity at a heat treatment time of 10 minutes in Test 3 is defined as 100%. In Fig. 6, four bar graphs are illustrated for each heat treatment time. The four bar graphs each represent the relative response sensitivity when the concentration of AQP is 0, 0.20%, 0.45%, or 1%, sequentially from the left.

Note that in Test 3, the measurement for one type of sample was performed twice using two samples of the same type. The results illustrated in Fig. 5 and Fig. 6 indicate the average values of the results at the two times of measurement.

From the results illustrated in Fig. 5 and Fig. 6, it was observed that when the concentration of AQP was 0 (without addition), the response sensitivity was reduced as the increase of heat treatment time, and the response sensitivity was almost lost at a heat treatment time of 60 minutes and 120 minutes. On the other hand, it was observed that when the concentration of AQP was 0.20%, the response sensitivity at any heat treatment time of 30 minutes, 60 minutes, or 120 minutes was lower than the response sensitivity at a heat treatment time of 10 minutes, but better than the response sensitivity when the concentration of AQP was 0. It was observed that when the concentration of AQP was 0.45%, the response sensitivity at a heat treatment time of 30 minutes, 60 minutes, and 120 minutes was almost the same as or higher than the response sensitivity at a heat treatment time of 10 minutes. It was further observed that when the concentration of AQP was 1%, the response sensitivity at any heat treatment time of 30 minutes, 60 minutes, and 120 minutes was two or more times higher than the response sensitivity at a heat treatment time of 10 minutes. From the above results, the upper limit concentration of polyaniline sulfonic acid in order for an enzyme electrode to obtain suitable heat resistance is 5% or less, preferably 1% or less, when the ease of preparation of the enzyme electrode is taken into consideration. Further, the lower limit concentration for obtaining suitable heat resistance is 0.01% or more, preferably 0.2% or more, more preferably 0.45% or more.

### <Test 4>

Next, the results of a test on the influence of the concentration of a conductive polymer added to a reagent solution will be described.

### <<Procedure for preparing enzyme electrode (glucose sensor)>>

The procedure for preparing a glucose sensor is as follows. Specifically, 0.1 µl of a mixed reagent was dropped on an electrode, allowed to stand for 20 minutes at room temperature, and then dried in an oven for 30 minutes at an oven temperature of 120°C.

### [Composition of mixed reagent ("%" is the percent by weight concentration)]

- Carbon black: 4%
- Aqueous Tween 20 solution: 1.6% (surfactant)
- Stabilizing agent: 0.5%
- Phosphate buffer (pH 7.0): 16 mM
- Aqueous glucose dehydrogenase (GDH) solution: 5.6 mg/ml
- Aqueous polyaniline sulfonic acid solution (0, 0.01%, 0.05%, 0.1%, 0.2%, 0.4%, 0.75%, 1%, 1.5%, and 2%)
- Water (H₂O)

### <<Evaluation procedure>>

As the electrodes of the glucose sensor prepared as described above, a silver/silver chloride electrode (Ag/AgCl (manufactured by BAS)) was used for the working electrode and the reference electrode, and a platinum (Pt) wire was used for the counter electrode. Such a glucose sensor was used to measure the glucose response current (response sensitivity) by amperometry in a PBS (Phosphate Buffered Saline) solution. Specifically, the applied voltage was set to +0.4 V (vs. Ag/AgCl), and a aqueous 2 M-glucose solution was added so that final concentration might be 100 mg/dL after background current reached a plateau in the PBS solution. A value obtained by subtracting the background current value from a recorded response current value after the addition of the aqueous glucose solution was defined as the response current value of the glucose sensor for the addition of the aqueous glucose solution.

### <<Results of Test 4>>

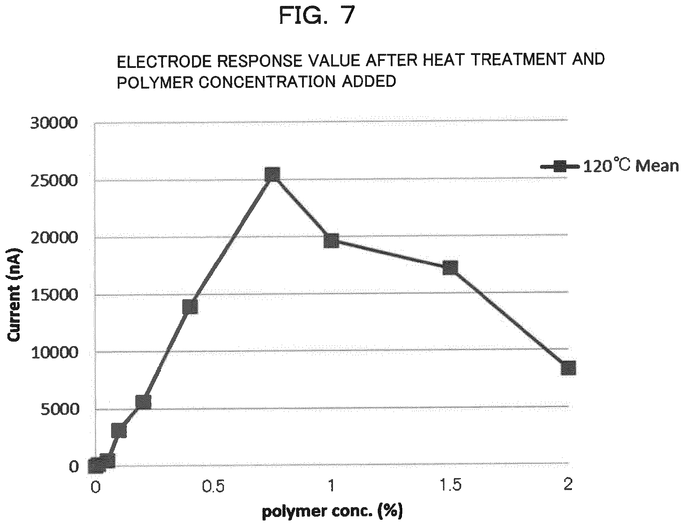

Fig. 7 is a graph illustrating the results of Test 4 obtained by the evaluation procedure as described above. Fig. 7 reveals that, at 120°C, suitable response current values are obtained over a concentration range of 0.1 to 2%. Generally, an enzyme is deactivated in high temperatures. On the other hand, since a suitable response current value is obtained even after the enzyme has passed through a high temperature of 120°C, it is found that the enzyme is properly protected from heat by polyaniline sulfonic acid. Further, it can also be grasped from the results illustrated in Fig. 7 that the concentration range that can be employed as the concentration of polyaniline sulfonic acid is 5% or less, preferably 2% or less, more preferably 1% or less, when the ease of preparation of the enzyme electrode is taken into consideration, and the lower limit concentration for obtaining suitable heat resistance is 0.01% or more, preferably 0.2% or more, more preferably 0.45% or more.

### <Test 5>

Next, the results of a test on the influence of heat treatment time of a glucose sensor will be described as Test 5. In Test 5, a plurality of glucose sensors were used, each of the sensors having been prepared by a procedure in which 0.1 µl of a mixed reagent having the composition as described with respect to Test 4 was dropped on the electrode, allowed to stand for 20 minutes at room temperature, and then subjected to heat treatment in an oven for any of a plurality of different drying times (heat treatment time of 10 minutes, 30 minutes, 60 minutes, or 120 minutes) at a predetermined oven temperature (100°C). The electrode configuration and evaluation procedure of the glucose sensor in Test 5 are the same as in Test 4.

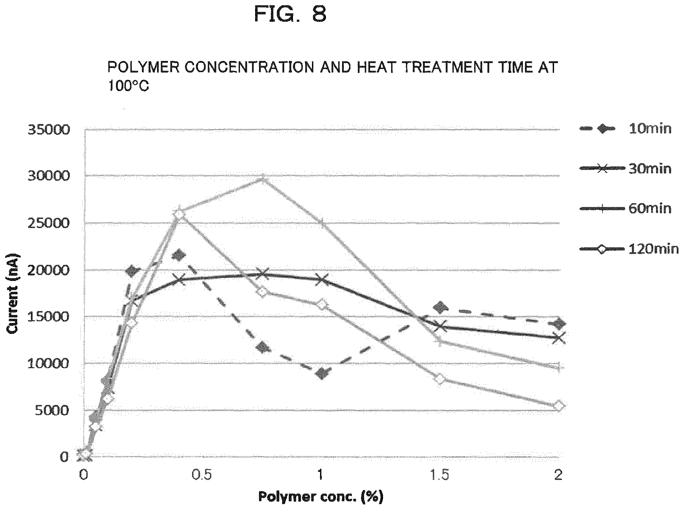

Fig. 8 is a graph illustrating the results of Test 5. Fig. 8 illustrates four graphs indicating the relationship between the polymer concentration and the response current value with respect to four heat treatment times (10 minutes, 30 minutes, 60 minutes, and 120 minutes). It is found that, in every polymer concentration, response current values which can be applied as a glucose sensor are obtained without the influence of heat treatment time. Therefore, it is found that the response sensitivity of the glucose sensor using polyaniline sulfonic acid is not largely influenced (proper heat resistance can be exhibited) even after the sensor is placed under a high temperature for a long time.

### [Operations and effects of the embodiment]

According to the enzyme electrode 10 as described above, suitable heat resistance can be obtained by forming a detection layer 2 containing oxidoreductase 4 and conductive polymer 5 (polyaniline sulfonic acid) on an electrode 1. Thereby, a reduction or loss in function of an enzyme or a reagent by heat can be suppressed. Thus, the life of the enzyme electrode 10 can be increased because the enzyme electrode 10 exhibits suitable heat resistance during each of the transportation, storage, and use thereof.

Note that the enzyme electrode 10 can be applied, for example, to a biosensor which is applied to glucose measurement as described above, or an electronic apparatus (for example, measuring device). Alternatively, the enzyme electrode 10 can also be applied as a part of an electric power unit that supplies, to a load, current generated by an enzyme reaction (current caused by electrons transferred to an electrode) as electric power through a feed section that connects the electrode and the load. The electronic apparatus can include an electronic apparatus including a biosensor to which the enzyme electrode according to the embodiment is applied and an electronic apparatus including the above device (electric power unit) to which the enzyme electrode according to the embodiment is applied. The components described in the embodiment as described above can be suitably combined within the scope of the invention as defined by the appended claims.

### Description of the reference numerals

1: electrode, 2: detection layer, 3: insulating substrate, 4: enzyme, 5: conductive polymer, 10: enzyme electrode

## Claims

1. An enzyme electrode (10) having improved heat resistance, comprising:
an electrode (1); and
a detection layer (2) that is in contact with the electrode and comprises an oxidoreductase (4), a water-soluble conductive polymer (5), and conductive particles,
wherein electrons are transferred between the enzyme and the electrode by direct electron transfer in the detection layer, and
wherein the oxidoreductase is a glucose dehydrogenase containing a cytochrome subunit; and wherein the water-soluble conductive polymer is polyaniline sulfonic acid and wherein the oxidoreductase is surrounded by the polymer.

2. The enzyme electrode according to claim 1, wherein the conductive particles comprise carbon.

3. The enzyme electrode according to claim 1 or 2, wherein said polyaniline sulfonic acid has been applied to said electrode in a solution at a concentration of 0.01 to 2% (by weight) and dried onto said electrode.

4. The enzyme electrode according to any one of claims 1 to 3, wherein a functional group of the polyaniline sulfonic acid is a hydroxy group or a sulfo group.

5. A biosensor, comprising:
an enzyme electrode (10) according to any one of claims 1 to 4.

6. An electronic apparatus comprising the biosensor according to claim 5.

7. An apparatus, comprising:
an enzyme electrode (10) according to any one of claims 1 to 4; and
a feed section configured to supply, to a load, a current generated by an enzyme reaction at the enzyme electrode.

8. An electronic apparatus comprising the apparatus according to claim 7.

9. A method for producing an enzyme electrode (10) having improved heat resistance, comprising:
forming a detection layer (2) on an electrode (1), the detection layer comprising an oxidoreductase (4), a water-soluble conductive polymer (5), and conductive particles, wherein the oxidoreductase is surrounded by the polymer, and wherein electrons are transferred between the enzyme and the electrode by direct electron transfer, and
wherein the oxidoreductase is a glucose dehydrogenase containing a cytochrome subunit; and
wherein the water-soluble conductive polymer is polyaniline sulfonic acid.

## Patentansprüche

1. Eine Enzymelektrode (10) aufweisend eine verbesserte Hitzebeständigkeit, umfassend:
eine Elektrode (1); und
eine Detektionsschicht (2), die mit der Elektrode in Kontakt steht und eine Oxidoreduktase (4), ein wasserlösliches leitfähiges Polymer (5) und leitfähige Partikel umfasst,
wobei Elektronen zwischen dem Enzym und der Elektrode durch direkten Elektronentransfer in der Detektionsschicht übertragen werden, und
wobei die Oxidoreduktase eine Glucose-Dehydrogenase ist, die eine Cytochrom-Untereinheit enthält; und
wobei das wasserlösliche leitfähige Polymer Polyanilinsulfonsäure ist und wobei die Oxidoreduktase von dem Polymer umgeben ist.

2. Enzymelektrode nach Anspruch 1, wobei die leitfähigen Partikel Kohlenstoff umfassen.

3. Enzymelektrode nach Anspruch 1 oder 2, wobei die Polyanilinsulfonsäure in einer Lösung in einer Konzentration von 0,01 bis 2 Gew.-% auf die Elektrode aufgebracht und auf der Elektrode getrocknet wurde.

4. Enzymelektrode nach einem der Ansprüche 1 bis 3, wobei eine funktionelle Gruppe der Polyanilinsulfonsäure eine Hydroxygruppe oder eine Sulfogruppe ist.

5. Biosensor, umfassend:
eine Enzymelektrode (10) nach einem der Ansprüche 1 bis 4.

6. Elektronische Einrichtung, die den Biosensor nach Anspruch 5 umfasst.

7. Einrichtung, umfassend:
eine Enzymelektrode (10) nach einem der Ansprüche 1 bis 4; und
einen Zuführungsabschnitt, der so konfiguriert ist, dass er einer Last einen durch eine Enzymreaktion an der Enzymelektrode erzeugten Strom zuführt.

8. Elektronische Einrichtung, die die Einrichtung nach Anspruch 7 umfasst.

9. Verfahren zur Herstellung einer Enzymelektrode (10) aufweisend eine verbesserte Hitzebeständigkeit, umfassend:
Bilden einer Detektionsschicht (2) auf einer Elektrode (1), wobei die Detektionsschicht eine Oxidoreduktase (4), ein wasserlösliches leitfähiges Polymer (5) und leitfähige Partikel umfasst, wobei die Oxidoreduktase von dem Polymer umgeben ist und wobei Elektronen zwischen dem Enzym und der Elektrode durch direkten Elektronentransfer übertragen werden, und
wobei die Oxidoreduktase eine Glucose-Dehydrogenase ist, die eine Cytochrom-Untereinheit enthält; und
wobei das wasserlösliche leitfähige Polymer Polyanilinsulfonsäure ist.

## Revendications

1. Électrode enzymatique (10) ayant une résistance à la chaleur améliorée, comprenant :
une électrode (1) ; et
une couche de détection (2) qui est en contact avec l'électrode et comprend une oxydoréductase (4), un polymère conducteur soluble dans l'eau (5) et des particules conductrices,
dans laquelle des électrons sont transférés entre l'enzyme et l'électrode par transfert direct d'électrons dans la couche de détection et
dans laquelle l'oxydoréductase est une déshydrogénase de glucose contenant une sous-unité de cytochrome ; et
dans laquelle le polymère conducteur soluble dans l'eau est de l'acide sulfonique de polyaniline et dans laquelle l'oxydoréductase est entourée par le polymère.

2. Electrode enzymatique selon la revendication 1, dans laquelle les particules conductrices comprennent du carbone.

3. Electrode enzymatique selon la revendication 1 ou 2, dans laquelle ledit acide sulfonique de polyaniline a été appliqué à ladite électrode dans une solution à une concentration de 0,01 à 2 % (en poids) et séché sur ladite électrode.

4. Electrode enzymatique selon l'une quelconque des revendications 1 à 3, dans laquelle un groupement fonctionnel de l'acide sulfonique de polyaniline est un groupement hydroxy ou un groupement sulfo.

5. Biocapteur comprenant :
une électrode enzymatique (10) selon l'une quelconque des revendications 1 à 4.

6. Appareil électronique comprenant le biocapteur selon la revendication 5.

7. Appareil comprenant :
une électrode enzymatique (10) selon l'une quelconque des revendications 1 à 4 ; et
une section d'alimentation configurée pour fournir, à une charge, un courant généré par une réaction enzymatique dans l'électrode enzymatique.

8. Appareil électronique comprenant l'appareil selon la revendication 7.

9. Procédé de production d'une électrode enzymatique (10) ayant une résistance à la chaleur améliorée, comprenant :
la formation d'une couche de détection (2) sur une électrode (1), la couche de détection comprenant une oxydoréductase (4), un polymère conducteur soluble dans l'eau (5) et des particules conductrices, dans lequel l'oxydoréductase est entourée par le polymère, et dans lequel des électrons sont transférés entre l'enzyme et l'électrode par transfert direct d'électrons, et
dans lequel l'oxydoréductase est une déshydrogénase de glucose contenant une sous-unité de cytochrome ; et
dans lequel le polymère conducteur soluble dans l'eau est de l'acide sulfonique de polyaniline.
